# EUROPEAN PATENT APPLICATION

(11) **EP 0 819 433 A2**
(43) Date of publication of application: **21.01.1998**
(21) Application number: 96120973.1
(22) Date of filing: 28.12.1996
(51) Int. Cl.: A61K 31/70, A61K 31/505

(54) **Compositions for increasing the efficacy of cancer drugs with tea catechin and/or theaflavin**

(30) Priority: 18.07.1996 JP 206361/96
(71) Applicant: CANCER INSTITUTE (HOSPITAL) CHINESE ACADAMY OF MEDICAL SCIENCES, Chaoyang Disctrict, Beijing 100021 (CH); INSTITUTE OF MEDICA BIOTECHNOLOGY, CHINESE ACADAMY OF MEDICAL SCIENCES, Beijing 100050 (CH); MITSUI NORIN CO., LTD., Tokyo (JP)
(72) Inventor: Cheng, Shu Jun, Chaoyang District, Beijing 100021 (JP); Wang, De Chang, Chaoyang District, Beijing 100021 (JP); Zhen, Yong Su, Tiantan, 100050, Beijing (JP); Nishino, Hoyoku, Hirakata-shi, Osaka-fu (JP); Hara, Yukihiko, Fujieda-shi, Shizuoka-ken (JP)
(74) Representative: Türk, Gille, Hrabal, Leifert

(57) **Abstract**

Method of strengthening an efficacy of cancer drug, especially anti-metabolite by administrating said cancer drug to a patient with tea catechins and/or theaflavins.

## Description

### FIELD OF THE INVENTION

The present invention relates to a method of increasing the efficacy of cancer drugs, more specifically it relates to a method of increasing the efficacy of cancer drugs by combining and administering tea catechins and/or theaflavins at the same time as the administration of the cancer drugs.

### BACKGROUND OF THE INVENTION

Cancer drugs administered to patients undergo oxidation in the body, and thus their effectiveness is reduced or eliminated. Also some types of cancer drugs produce active oxygen during metabolic processes and this can cause damage to the tissue of normal organs, causing some patients to suffer greatly from the side-effects.

With these points in mind we conducted research into a method of increasing the efficacy of cancer drugs, reducing side-effects thereof.

One of such methods which has been investigated is to administer an antioxidant at the same time as the administration of the cancer drug. However, up until now these attempts could not have been said to have reached a sufficient level of success.

### SUMMARY OF THE INVENTION

The present inventors conducted extensive research into tea polyphenols such as tea catechins and theaflavins which are the dimers of catechins, and have reported that tea polyphenols have a strong antioxidative action, and free radical scavenging action.

As a result of extensive research into the applications of these functions of tea polyphenols, it was discovered that according to the administration of tea polyphenols at the same time as the administration of an anti-metabolite which is one type of cancer drug, the action of the cancer drug is strengthened, and thus the present invention was completed.

That is to say, the present invention relates to a method of increasing the efficacy of cancer drug by the addition of tea catechins and/or theaflavins to the cancer drug.

### DETAILED DESCRIPTION OF THE INVENTION

The cancer drugs which are applicable to the method of the present invention are not restricted, and in particular chemical cancer drugs are given as preferable examples, but amongst these anti-metabolites are most suitable. Anti-metabolites are compounds which act antagonistically on the metabolism of purine base or pyrimidine base, such as cytarabine, methotrexate, 6-mercaptopurine, 5-fluorouracil etc., but among these cytarabine (1-β-D-arabinofuranosylcytosine: AraC) or methotrexate (4-amino-4-deoxy-10-methylfolic acid: MTX) are suitable.

The tea polyphenols used in the present invention may be tea catechins or theaflavins obtained by extraction of green tea leaves, black tea leaves etc. with water or organic solvents such as low-grade alcohol, acetone, ethyl acetate, and these may be further separated, purified and used individually or two or more may be combined and used.

Tea catechins are of the general formula I as shown below. 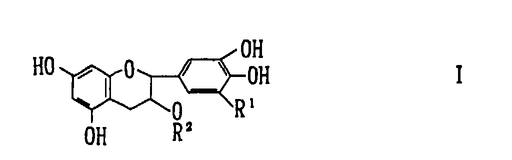 (where R¹ represents H or OH, and R² represents H or

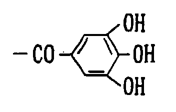

Specifically tea catechins include epicatechin, epicatechin gallate, epigallocatechin, epigallocatechin gallate, gallocatechin etc. (and their derivatives) and these may be used separately or a combination of two or more may be used. Among these, it is desirable that at least one of the following: epigallocatechin gallate, epicatechin gallate and epigallocatechin, is used. In particular it is desirable that a tea catechin wherein epigallocatechin gallate is a main catechin component; as in for example, Polyphenon 100™ (Mitsui Norin Co., Ltd., Composition: (-)epicatechin, 10.8%, (-)epigallocatechin 9.2%, (-)epicatechin gallate 6.5%, (-)epigallocatechin gallate 54.8%, (-) gallocatechin gallate 4.0%) or Polyphenon 60™ (Mitsui Norin Co., Ltd., Composition: (-)epigallocatechin 21.0%, (-)epicatechin, 7.3%, (-) epigallocatechin gallate 29.2%, (-)epicatechin gallate 7.9%)

Theaflavins used in the present invention are of the general formula II as shown below. 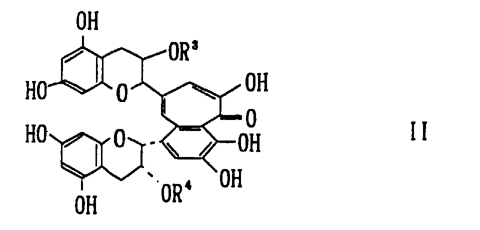 (where R³ and R⁴ represents H or 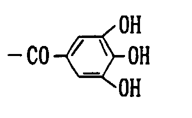 and R³ and R⁴ may be the same or different).

Specifically, theaflavins include free theaflavins, theaflavin monogallate A, theaflavin monogallate B, theaflavin digallate etc. (and their derivatives), and these may be used individually, or two or more may be combined and used. In particular, desirable is a theaflavin containing theaflavin digallate as a main component; as in for example, Polyphenon TF™ (Mitsui Norin Co., Ltd., Composition: theaflavin 16.8%, theaflavin monogallate A 19.5%, theaflavin monogallate B 16.1%, theaflavin digallate 31.4%) may be used.

According to the present invention, the efficacy of cancer drugs can be strengthened by the addition of tea catechins and/or theaflavins at the same time as the administration of the above-mentioned cancer drugs.

The ratio of the cancer drug to the tea catechin and/or theaflavin should be decided according to the purpose of use, the method of administration of the drug and the type of cancer drug etc., but generally an amount of the cancer drug should be 1-20 mg/kg in case of intraperitoneal administration (i.p.), or more desirably, 5-15 mg/kg (i.p.), and an amount of the tea catechin and/or theaflavin should be 1-30 mg/kg in case of intraperitoneal administration (i.p,), or more desirably, 5-20 mg/kg (i.p.) or 100-1000 mg/kg in case of oral administration (p.o.).

According to the method of the present invention, to increase the efficacy of cancer drugs, tea catechins and/or theaflavins should be administered to patients in the ratios as described above. This mixture can be administered to patients in various forms. For example, it may be combined with a suitable excipient and can be used in an oral and/or non-oral form. A suitable amount of an auxiliary compound such as a lubricant, an emulsifier, a dispersing agent may be used.

As an oral administration, it may be in the form of a liquid, powder, tablet, capsule, granules etc., and in these cases the excipient used apart from water, could be sugars, starch, dextran, calcium phosphate, calcium carbonate, magnesium oxide, magnesium stearate, aluminium silicate, aluminium hydroxide, sodium carbonate, glycerol etc.

As a non-oral administration, it may be in the form of an injection, a drip, and ointment, and it could be mixed with suitable substances such as distilled water, physiological salt, plant oils such as olive oil, alcohol such as ethanol, and polyethylene glycol.

According to the method of the present invention, the efficacy of cancer drugs is strengthened without any side-effects since the cancer drug, in particular an anti-metabolite is used in combination with tea catechins and/or theaflavins.

### EXAMPLES

The present invention will be explained by reference to the following examples.

### Example 1:

1.5 x 10⁶ Leukemia L-1210 tumor cells were administered intraperitoneally to Balb/c mice (18-20 g), the mice were divided into 6 groups (10 mice in each group) and after 24 hours a fixed amount of each type of drug was intraperitoneally administered once a day for a period of seven days.

The average rate of survival of each group and the rate of survival of the drug administered group as compared to the control (T/C(%)) as well as long survival rate (over 60 days) were calculated. The results are shown in Table 1.

**Table 1**

| Group | Dose (mg/kg) | Survival days average (day)±SD | T/C (%) | Number of survivors (over 60 days) |
|---|---|---|---|---|
| Control | ― | 16.1± 1.1 | | 0/10 |
| AraC | 10 | 28.7±11.8 | 178 | 1/10 |
| EGCg | 10 | 26.8± 4.5 | 166 | 0/10 |
| AraC + EGCg | 10+10 | 60.0± 0 | 373 * | 10/10 |
| EGCg | 20 | 12.8± 3.7 | 80 | 0/10 |
| AraC + EGCg | 10+20 | 37.3±20.1 | 232 * | 4/10 |

| | | | | |
|---|---|---|---|---|
| * P <0.01 | | | | |
| SD: significant difference | | | | |

As is evident from the table, administration of a combination of cytarabine (AraC) and epigallocatechin gallate (EGCg) significantly increased the survival rate of the mice, and moreover there was a high survival rate over 60 days. No side-effects were noticed.

### Example 2:

This example was conducted in the same way as in Example 1 except that the amount of EGCg added was different. Results, as shown in Table 2, show that in this case too, the administration of a combination of cytarabine and epigallocatechin gallate increased the survival rate of the mice and significantly increased the survival rate over 60 days.

**Table 2**

| Group | Dose (mg/kg) | Survival days average (day)±SD | T/C (%) | Number of survivors (over 60 days) |
|---|---|---|---|---|
| Control | ― | 17.8± 1.2 | | 0/10 |
| AraC | 10 | 35.3±17.3 | 176 | 2/10 |
| EGCg | 5 | 21.0± 5.2 | 118 | 0/10 |
| AraC + EGCg | 10+5 | 49.2±17.7 | 275 * | 7/10 |
| EGCg | 10 | 21.4± 4.7 | 120 | 0/10 |
| AraC + EGCg | 10+10 | 60.0± 0 | 337 * | 10/10 |

| | | | | |
|---|---|---|---|---|
| * P <0.01 | | | | |
| SD: significant difference | | | | |

### Example 3:

This example was carried out in the same way as Example 1 except that the tumor cells used were Leukemia P388. Results are shown in Table 3. As is evident from the table, the survival rate of the mice increased and the survival rate over 60 days was significantly higher according to the administration of a combination of cytarabine and epigallocatechin gallate.

**Table 3**

| Group | Dose (mg/kg) | Survival days average (day)±SD | T/C (%) | Number of survivors (over 60 days) |
|---|---|---|---|---|
| Control | ― | 15.4 ± 0.7 | | 0/10 |
| AraC | 10 | 18.9 ± 1.4 | 123 | 0/10 |
| EGCg | 5 | 18.2 ± 1.3 | 118 | 0/10 |
| AraC + EGCg | 10+5 | 35.2 ±21.3 | 229 * | 4/10 |
| EGCg | 10 | 17.7 ± 1.8 | 115 | 0/10 |
| AraC + EGCg | 10+10 | 60.0 ± 0 | 390 * | 10/10 |

| | | | | |
|---|---|---|---|---|
| * P <0.01 | | | | |
| SD: significant difference | | | | |

### Example 4:

KM mice (26-28 g) were each innoculated subcutaneously with 5 x 10⁶ Sarcoma tumor cells (S-180), the mice were divided into 5 groups (10 mice in each group), and after 24 hours each drug was administered once a day for a period of seven days. According to the intraperitoneal administration 3 mg/kg of methotrexate (MTX) was administered, and 230 mg/kg or 460 mg/kg of epigallocatechin gallate (EGCg) was administered orally.

24 hours after administration of the last drug, the mice were killed, the weight of the tumors was calculated, and according to the extent of metabolic damage the efficacy of the drugs was calcutated. Results are shown in Table 4. As is evident from the table, the weight of the tumors was significantly decreased by administration of a combination of methotrexate and epigallocatechin gallate.

**Table 4**

| Group | Number of mice | Dose mg/kg | Tumor weight g± SD | Inhibition (%) | P value |
|---|---|---|---|---|---|
| Control | 10 | ― | 2.13±0.58 | ― | ― |
| MTX | 10 | 3.0 i.p. ×7 | 1.00±0.18 | 53.1 | <0.01^{#} |
| EGCg | 10 | 230 p.o. ×7 | 1.76±0.72 | 17.4 | >0.05^{#} |
| MTX + EGCg | 10 | 3.0 i.p. ×7 | 0.93±0.31 | 56.3 | >0.05* |
| | | 230 p.o. ×7 | | | |
| MTX + EGCg | 10 | 3.0 i.p. ×7 | 0.82±0.46 | 61.5 | >0.05* |
| | | 460 p.o. ×7 | | | |

| | | | | | |
|---|---|---|---|---|---|
| #: compare with control | | | | | |
| *: compare with MTX | | | | | |
| i.p. : intraperitoneal administration p.o. : oral administration SD: significant difference | | | | | |

## Claims

1. A method of strengthening an efficacy of cancer drug which comprises administrating said cancer drug to a patient with tea catechin and/or theaflavin.

2. The method of Claim 1 wherein the cancer drug is an antimetabolite.

3. The method of Claim 1 wherein the cancer drug is cytarabine or methotrexate.

4. The method of Claim 1 wherein the tea catechin consists of epigallocatechin gallate as a main component.

5. The method of Claim 1 wherein the tea catechin contains at least one of the following: epigallocatechin gallate, epicatechin gallate, and epigallocatechin.

6. The method of Claim 1 wherein the theaflavin consists of theaflavin digallate as a main component.

7. A cancer drug composition having improved efficacy which consists essentially of cancer drug and tea catechin and/or theaflavin.

8. The cancer drug of Claim 7 wherein the cancer drug is in an amount of 1-20 mg/kg in case of intraperitoneal administration and the tea catechin is in an amount of 5-20 mg/kg in case of intraperitoneal administration.

9. The cancer drug of Claim 7 wherein the cancer drug is in an amount of 1-20 mg/kg in case of intraperitoneal administration and the theaflavin is in an amount of 5-20 mg/kg in case of intraperitoneal administration.

10. The cancer drug of Claim 7 wherein the cancer drug is in an amount of 1-20 mg/kg in case of intraperitoneal administration and the tea catechin and/or theaflavin is in an amount of 100-1000 mg/kg in case of oral administration.
